# EUROPÄISCHE PATENTSCHRIFT

(11) **EP 1 090 028 B1**
(45) Veröffentlichungstag und Bekanntmachung des Hinweises auf die Patenterteilung: **23.01.2002**
(21) Anmeldenummer: 99938195.7
(22) Anmeldetag: 18.06.1999
(51) Int. Cl.: C07J 53/00, A61K 31/565

(54) **UNGESÄTTIGTE 14,15-CYCLOPROPANO-ANDROSTANE, VERFAHREN ZU IHRER HERSTELLUNG UND DIESE VERBINDUNGEN ENTHALTENDE PHARMAZEUTISCHE PRÄPARATE**
UNSATURATED 14,15-CYCLOPROPANOANDROSTANES, METHOD FOR THE PRODUCTION THEREOF AND PHARMACEUTICAL PREPARATIONS CONTAINING SAID COMPOUNDS
14,15-CYCLOPROPANOANDROSTANES INSATURES, LEUR PROCEDE DE PRODUCTION ET PREPARATIONS PHARMACEUTIQUES CONTENANT CES COMPOSES

(30) Priorität: 22.06.1998 DE 19827523
(43) Veröffentlichungstag der Anmeldung: 11.04.2001
(73) Patentinhaber: Jenapharm GmbH & Co. KG, 07745 Jena (DE)
(72) Erfinder: RING, Sven, D-07749 Jena (DE); SCHWARZ, Sigfrid, D-07743 Jena (DE); ELGER, Walter, D-14195 Berlin (DE); SCHNEIDER, Birgitt, D-07745 Jena (DE); KAUFMANN, Günter, D-07743 Jena (DE); SOBEK, Lothar, D-07743 Jena (DE)
(74) Vertreter: Cramer, Eva-Maria
(86) Internationale Anmeldenummer: DE9901794
(87) Internationale Veröffentlichungsnummer: WO9967275

(56) Entgegenhaltungen:
- EP-A- 0 768 316
- DD-A- 293 591
- US-A- 3 007 945
- US-A- 3 194 803
- US-A- 3 262 949
- US-A- 3 624 111
- US-A- 4 100 027

## Beschreibung

Die Erfindung betrifft neue ungesättigte 14,15-Cyclopropano-Androstane, deren Herstellung und diese Verbindungen enthaltende pharmazeutische Präparate.

Aus EP 0 768 316 A1 sind Steroide mit einer 14,15-Methylengruppe bekannt, die progesterone Wirkung aufweisen und damit in Kombination mit mindestens einem geeigneten Estrogen zur hormonalen Kontrazeption und in der klimakterischen Hormon-Replacement-Therapie (HRT) sowie zur Behandlung der Endometriose oder gestagenabhängiger Tumore geeignet sind.

Die vorlieginde Erfindung betrifft ungesättigte 14,15-Cyclopropano-Androstane der allgemeinen Formel I

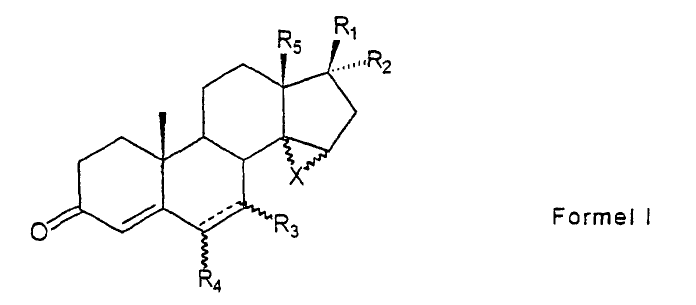

In der allgemeinen Formel I ist R; ein Wasserstoffatom, eine Hydroxygruppe, eine Alkyloxy-, Acyioxy-, Aryloxy-, Aralkyloxy- oder eine Alkylaryloxygruppe , ein Rest -OCONHR₉ₐ oder -OCOOR₉ₐ mit R₉ₐ für ein Wasserstoffatom , einen Alkyl-, Aryl- , Aralkyl- oder Alkylarylrest mit jeweils 1-10 Kohlenstoffatomen,
steht R₂ für ein Wasserstoffatom, eine Hydroxylgruppe eine Alkyl-, Acyl-, Aryl-, Aralkyl-, Alkylarylgruppe mit jeweils 1-10 Kohlenstoffatomen,
für einen Rest -(CH₂)ₙCH₂Y mit n = 0, 1 oder 2 und Y für ein Fluor-, Chlor-, Brom-, oder Jodatom , für eine Cyano-, Azido- oder Rhodanogruppe, für einen Rest -OR₁₀ oder -SR₁₀ mit R₁₀ für ein Wasserstoffatom, einen Alkyl-, Aryl-, Aralkyl- oder Alkylarylrest mit jeweils 1-10 Kohlenstoffatomen oder einen Acylrest COR_{9b} mit R_{9b} für einen Alkyl-, Aryl-. Aralkyl- oder Alkylarylrest mit jeweils 1-10 Kohlenstoffatomen,
einen Rest -OR₉ₐ mit R₉ₐ für ein Wasserstoffatom, ein Alkyl-, Aryl-, Aralkyl- oder Alkylarylrest mit jeweils 1 - 10 Kohlenstoffatomen ,
für einen Rest - (CH₂)ₘ-CH=CH(CH₂)ₙ-R₈ mit m =0, 1, 2 oder 3 und n = 0, 1 oder 2 ist und R₈ für ein Wasserstoffatom, einen Alkyl-, Aryl-, Aralkyl- oder Alkylarylrest mit jeweils 1-10 Kohlenstoffatomen oder eine Hydroxylgruppe, eine Alkoxygruppe oder Acyloxygruppe mit jeweils 1-10 Kohlenstoffatomen,
einen Rest - (CH₂)ₒC=-CR₁₁ mit o = 0,1 oder 2 und R₁₁ für ein Wasserstoffatom, ein Fluor-, Chlor-, Brom- oder lodatom, einen Alkyl-, ArylAralkyl-, Alkylaryl- oder einen Acylrest mit jeweils 1-10 Kohlenstoffatomen,
stehen R₁ und R₂ für eine Keto-, Methylen-, Difluormethylengruppe,
kann sich zwischen C-6 und C-7 eine Doppelbindung befinden,
befindet sich zwischen C-14 und C-15 eine α- oder β- Cyclopropangruppe X mit X für eine CZ₂- Gruppe mit Z für ein Wasserstoff-, Fluor-, Chlor-, Brom- oder lodatom,
stehen R₃ und R₄ unabhängig voneinander für ein Wasserstoffatom, eine α- oder β- ständige Alkylgruppe mit 1-10 Kohlenstoffatomen
steht R₅ für eine Alkylgruppe mit 1-3 Kohlenstoffatomen.

Die erfindungsgemäßen Verbindungen, die neuen ungesättigten 14,15-Cyclopropano-Androstane, sind bisher nicht beschrieben.

Ihre biologische Wirkung ist noch nicht bekannt.

Des weiteren betrifft die vorliegende Erfindung pharmazeutische Präparate, die mindestens eine Verbindung der allgemeinen Formel I oder deren pharmazeutisch annehmbare Salze enthalten.

In der allgemeinen Formel I 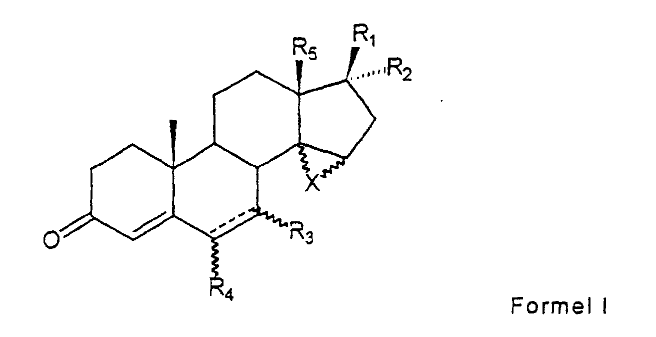 ist R₁ ein Wasserstoffatom, eine Hydroxygruppe, eine Alkyloxy-, Acyloxy-, Aryloxy-, Aralkyloxy- oder eine Alkylaryloxygruppe , ein Rest-OCONHR₉ₐ oder -OCOOR₉ₐ mit R₉ₐ für ein Wasserstoffatom , einen Alkyl-, Aryl- , Aralkyl- oder Alkylarylrest mit jeweils 1-10 Kohlenstoffatomen,
steht R₂ für ein Wasserstoffatom, eine Hydroxylgruppe eine Alkyl-, Acyl-, Aryl-, Aralkyl-, Alkylarylgruppe mit jeweils 1-10 Kohlenstoffatomen,
für einen Rest -(CH₂)ₙCH₂Y mit n = 0, 1 oder 2 und Y für ein Fluor-, Chlor-, Brom-, oder Jodatom , für eine Cyano-, Azido- oder Rhodanogruppe, für einen Rest -OR₁₀ oder -SR₁₀ mit R₁₀ für ein Wasserstoffatom, einen Alkyl-, Aryl-, Aralkyl- oder Alkylarylrest mit jeweils 1-10 Kohlenstoffatomen oder einen Acylrest COR_{9b} mit für einen Alkyl-, Aryl-, Aralkyl- oder Alkylarylrest mit jeweils 1-10 Kohlenstoffatomen, einen Rest -OR₉ₐ mit R₉ₐ für ein Wasserstoffatom, ein Alkyl-, Aryl-, Aralkyl- oder Alkylarylrest mit jeweils 1 - 10 Kohlenstoffatomen ,
für einen Rest - (CH₂)ₘ-CH=CH(CH₂)ₙ-R₈ mit m =0, 1, 2 oder 3 und n = 0, 1 oder 2 ist und R₈ für ein Wasserstoffatom, einen Alkyl-, Aryl-, Aralkyl- oder Alkylarylrest mit jeweils 1-10 Kohlenstoffatomen oder eine Hydroxylgruppe, eine Alkoxygruppe oder Acyloxygruppe mit jeweils 1-10 Kohlenstoffatomen,
einen Rest - (CH₂)ₒC≡CR₁₁ mit o = 0,1 oder 2 und R₁₁ für ein Wasserstoffatom, ein Fluor-, Chlor-, Brom- oder lodatom, einen Alkyl-, Aryl-, Aralkyl-, Alkylaryl- oder einen Acylrest mit jeweils 1-10 Kohlenstoffatomen,
steht R₁ und R₂ für eine Keto-, Methylen-, Difluormethylengruppe,
kann sich zwischen C-6 und C-7 eine Doppelbindung befinden,
befindet sich zwischen C-14 und C-15 eine α- oder β- Cyclopropangruppe X mit X für eine CZ₂- Gruppe mit Z für ein Wasserstoff-, Fluor-, Chlor-, Brom- oder lodatom,
stehen R₃ und R₄ unabhängig voneinander für ein Wasserstoffatom, eine α- oder β- ständige Alkylgruppe mit 1-10 Kohlenstoffatomen,
steht R₅ für eine Alkylgruppe mit 1-3 Kohlenstoffatomen.

Am meisten bevorzugt sind
17β-Hydroxy-14α,15α-methylen-androst-4-en-3-on (J 1193),
17α-Hydroxy-14α,15α-methylen-androst-4-en-3-on,
17β-Hydroxy-14β,15β-methylen-androst-4-en-3-on,
17α-Hydroxy-14β,15β-methylen-androst-4-en-3-on,
17α-Methyl,17β-Hydroxy-14α,15α-methylen-androst-4-en-3-on,
17β-Methyl,17α-Hydroxy-14α,15α-methylen-androst-4-en-3-on;
17α-Methyl,17β-Hydroxy-14β,15β-methylen-androst-4-en-3-on,
17β-Methyl,17α-Hydroxy-14β,15β-methylen-androst-4-en-3-on,
17β-Hydroxy-6α-Methyl-14α,15α-methylen-androst-4-en-3-on,
17α-Hydroxy-6α-Methyl-14α,15α-methylen-androst-4-en-3-on,
17β-Hydroxy-6α-Methyl-14β,15β-methylen-androst-4-en-3-on,
17α-Hydroxy-6α-Methyl-14β,15β-methylen-androst-4-en-3-on,
17β-Hydroxy-7α-Methyl-14α,15α-methylen-androst-4-en-3-on,
17α-Hydroxy-7α-Methyl-14α,15α-methylen-androst-4-en-3-on,
17β-Hydroxy-7α-Methyl-14β,15β-methylen-androst-4-en-3-on,
17α-hydroxy-7α-Methyl-14β,15β-methylen-ancrost-4-en-3-on.
17β-Hydroxy-14α,15α-methylen-androsta-4,6-dien-3-on,
17α-Hydroxy-14α,15α-methylen-androsta-4,6-dien-3-on,
17β-Hydroxy-14β,15β-methylen-androsta-4,6-dien-3-on,
17α-Hydroxy-14β,15β-methylen-androsta-4,6-dien-3-on,
17α-Methyl,17β-Hydroxy-14α,15α-methylen-androsta-4,6-dien-3-on,
17β-Methyl,17α-Hydroxy-14α,15α-methylen-androsta-4,6-dien-3-on,
17α-Methyl,17β-Hydroxy-14β,15β-methylen-androsta-4,6-dien-3-on,
17β-Methyl,17α-Hydroxy-14β,15β-methylen-androsta-4,6-dien-3-on,
17β-Hydroxy-7α,17α-Dimethyl-14α,15α-methylen-androst-4-en-3-on,
17α-Hydroxy-7α,17β-Dimethyl-14α,15α-methylen-androst-4-en-3-on,
17β-Hydroxy-7α,17α-Dimethyl-14β,15β-methylen-androst-4-en-3-on,
17α-Hydroxy-7α,17β-Dimethyl-14β,15β-methylen-androst-4-en-3-on,

Die Erfindung betrifft ferner ein Verfahren zur Herstellung der Verbindungen nach der allgemeinen Formel I und deren pharmazeutisch annehmbaren Salze, das dadurch gekennzeichnet ist, daß man eine Verbindung der allgemeinen Formel II, 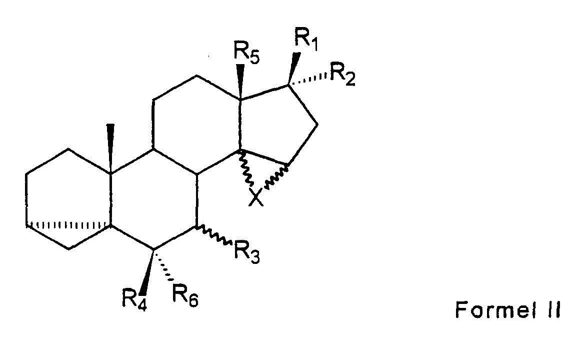 worin R₁, R₂, R₃ und R₅ die vorstehend angegebene Bedeutung besitzen,
R₄ für ein Wasserstoffatom, eine Hydroxygruppe , eine Alkoxylgruµpe, eine Acyloxygruppe, eine Alkylgruppe mit jeweils 1-10 Kohlenstoffatomen steht,
R₆ für ein Wasserstoffatom, eine Hydroxygruppe , eine Alkoxylgruµpe, eine Acyloxygruppe , eine Alkylgruppe mit jeweils 1-10 Kohlenstoffatomen steht,
sich zwischen Kohlenstoffatom 14 und 15 eine α- oder β- Cyclopropangruppe X befindet mit X für eine CZ₂- Gruppe darstellt, worin Z ein Wasserstoff-, Fluor-, Chlor-, Brom- oder lodatom bedeuten kann,
die 3,5-Cyclopropangruppierung mittels Säuren, wobei Mineralsäuren, organische Säuren und Lewissäuren bevorzugt sind, spaltet und danach nach den dem Fachmann bekannten Methoden in die gewünschten Verbindungen der allgemeinen Formel I überführt.

Gegenstand der vorliegenden Erfindung sind Arzneistoffe zur oralen, rektalen, subcutanen, intravenösen oder intramuskulären Anwendung, die zusammen mit den üblichen Trägerstoffen und Verdünnungsmitteln mindestens eine Verbindung der allgemeinen Formel I oder deren Säureadditionssalze als Wirkstoff enthalten.

Pharmazeutische Präparate der Erfindung werden mit den üblichen festen oder flüssigen Trägerstoffen und/oder Verdünnungsmitteln und den allgemein üblicherweise verwendeten Hilfsstoffen entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver,Lösungen oder Suspensionen auch als Depotform zubereitet.

Daneben sind parenterale Arzneiformen wie Injektionslösungen oder aber Suppositorien in Betracht zu ziehen.

Arzneiformen als Tabletten können beispielsweise durch Mischen des Wirkstoffes mit bekannten Hilfsstoffen, wie Dextrose. Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.

Analog lassen sich Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid, oder Zucker bereiten. Die Drageehülle kann dabei auch aus mehreren Schichten bestehen, wobei beispielsweise oben genannte Hilfsstoffe verwendet werden.

Die Lösungen oder Suspensionen mit dem erfindungsgemäßen Wirkstoff können zur Verbesserung des Geschmacks mit Stoffen wie Saccharin, Cyclamat oder Zucker und / oder mit Aromastoffen, wie Vanillin oder Orangenextrakt versetzt werden. Weiterhin können sie mit Suspendierhilfsstoffen wie Natriumcarboxymethylcellulose oder Konservierungsmitteln wie p-Hydroxybenzoesäure vermischt werden.

Die Bereitung von Kapseln kann durch Mischen des Arzneistoffes mit Trägern wie Milchzucker oder Sorbit erfolgen, die dann in die Kapseln eingebracht werden.

Die Herstellung von Suppositorien erfolgt vorzugsweise durch Mischung des Wirkstoffes mit geeigneten Trägermaterialien wie Neutralfetten oder Polyethylenglykolen oder dessen Derivaten.

Bei den pharmazeutischen Zubereitungsformen kann es sich weiterhin um perkutane Zubereitungsformen, z.B. Transdermale Therapeutische Systeme (TTS) oder Gele, Sprays oder Salben oder um intranasale Zubereitungsformen wie Nasenspray oder Nasentropfen handeln.

Die erfindungsgemäßen ungesättigten 14,15-Cyclopropano-Androstane der allgemeinen Formel I sind hormonell (gestagen und/oder androgen wirkende) Verbindungen. So ist beispielsweise die Verbindung der allgemeinen Formel I, in der R₁ eine Hydroxylgruppe, R₂, R₃, R₄) ein Wasserstoffatom, R₅ eine Methylgruµpe , X eine CH₂-Gruppe darstellt und der 14,15-Cyclopropanring α- ständig ist, 17β-Hydroxy-14α,15α-methylen-androst-4-en-3-on (J 1193), ein Androgen.

Während die Substanz 17β-Hydroxy-14α,15α-methylen-androst-4-en-3-on (J 1193) mit 24 ± 3 % an den Androgenrezeptor der Rattenprostata (Referenzsubstanz: 17β-Hydroxy-17α-methyl-estra- 4,9,11-trien-3-on; R 1881) bindet, besteht praktisch keine Bindung an den Progesteronrezeptor des Kaninchenuterus (Referenzsubstanz: Progesteron). Im Hershberger-Test konnte eine deutliche androgene Aktivität nachgewiesen werden, demgegenüber ist die gestagene Wirkung im Graviditätserhaltungstest kaum vorhanden. Die Substanz 17β-Hydroxy-14α,15α-methylen-androst-4-en-3-on (J 1193) zeigt ein reines androgenes Wirkprofil, nahezu frei von gestagenen Eigenschaften.

Diese Testergebnisse eröffnen den erfindungsgemäßen Verbindungen der allgemeinen Formel I vielfältige Möglichkeiten für die Fertilitätskontrolle beim Mann , die Hormonreplacement-Therapie bei Mann und

Frau oder die Behandlung hormonell bedingter Erkrankungen bei Mann und Frau , wie beispielsweise Endometriose, Mammacarcinom oder Hypogonadismus.

Die erfindungsgemäßen Verbindungen der allgemeinen Formel I sollen an den nachfolgenden Beispielen näher erläutert, jedoch nicht eingeschränkt werden.

### Beispiel 1

### 17β-Hydroxy-14α,15α-methylen-androst-4-en-3-on

1 g 17β-Acetoxy-3,5-cyclo-6β-methoxy-14α,15α-methylen-androstan wird in 50 ml Aceton gelöst , mit 0,1 ml 60 % iger Perchlorsäure versetzt und 40 min bei 45 °C gerührt. Es werden 20 ml Wasser zugegeben und danach neutralisiert man mit Natriumhydrogencarbonat. Das Lösungsmittel wird abgezogen , das kristalline Produkt wird abgesaugt und in 200 ml. Toluol aufgenommen. Von dieser Lösung werden 50 ml abdestilliert , danach werden 150 mg Aluminiumisopropylat und 2 ml Cyclohexanon zugegeben und es wird 40 min auf 90 °C erhitzt. Man läßt abkühlen, säuert mit 1 n Salzsäure an und arbeitet extraktiv auf. Der organische Extrakt wird eingeengt und der Rückstand wird mit 100 ml 1n methanolischer Kaliumhydroxidlösung 60 min am Rückfluss erhitzt. Nach dem Abkühlen wird mit 1 n Salzsäure neutralisiert und das Lösungsmittel wird abgezogen , wobei das Produkt fest ausfällt. Das Kristallisat wird abgesaugt und mit Wasser gewaschen . Der Rückstand wird an Kieselgel (Laufmittel : Toluol/Essigester 10:2) chromatografiert und aus Essigester umkristallisiert.
Fp. 167-172 °C; H-NMR: 0,13 (1H, dd, J=5.6, 3.2 Hz, CH₂-bridge) 0,24 (1H, dd, J=8.3, 5.6 Hz, CH₂-bridge) 1,01 (3H, s, H-18) 1,26 (3H, s, H-19) 3,49 (1H, dd, J=9.4, 6.6 Hz, H-17) 5,71 (1H, s, H-4)

### Beispiel 2

### 17β-Hydroxy-14β,15β-methylen-androst-4-en-3-on

Die Substanz wird aus 17β-Acetoxy-3,5-cyclo-6β-methoxy-14β,15β-methylen-androstan analog der Vorschrift in Beispiel 1 hergestellt.

Fp. 214-216 °C; H-NMR: 0,52 (1H, dd, J=8.3, 4.9 Hz, CH₂-bridge) 0,67 (1H, dd, J=4.9, 3.8 Hz, CH₂-bridge) 1,09 (3H, s, H-18) 1,20 (3H, s, H-19) 3,62 (1H, d, J=6.3 Hz, H-17) 5,71 (1H, s, H-4)

### Beispiel 3

### 17β-Hydroxy-14β,15β-methylen-androsta-4,6-dien-3-on

1g 17β-Hydroxy-14β,15β-methylen-androst-4-en-3-on (Herstellung Beispiel 2) wird mit 1,2 g Chloranil in 50 ml tert.-Butanol 30 min am Rückfluss gekocht. Man läßt abkühlen und engt zur Trockene ein . Der Rückstand wird an Kieselgel (Laufmittel : Dichlormethan/Essigester 10:1) chromatografiert. Zur weiteren Reinigung wird aus Essigester umkristallisiert.
Fp. 180-190 °C; H-NMR: 0,8 (2H, m, CH₂-bridge) 1,12 (3H, s, H-18) 1,13(3H, s, H-19) 3,65 (1H,d, J=6.2 Hz, H-17) 5,67 (1H, s, H-4) 5,95 (2H, m, H-1,H-2)

### Beispiel 4

### 17β-Hydroxy-7α-Methyl-14β,15β-methylen-androst-4-en-3-on

Zu einer Lösung von Methylmagesiumiodid (bereitet aus 2,5 g Magnesium und 6,4 ml Methyliodid in 80 ml Diethylether) werden 80 ml THF gegeben , man kühlt auf -5°C und gibt 1 g Kupferacetat- monohydrat ,gelöst in 50 ml THF , zu. Es wird auf -20°C gekühlt und dann wird eine Lösung von 5 g 17β-Hydroxy-14β,15β-methylen-androsta-4,6-dien-3-on in 80 ml THF zugetropft. Nach 2 Stunden gießt man auf Eiswasser/ 2N Schwefelsäure und extrahiert mit 3 mal 80 ml Essigester. Der organische Extrakt wird getrocknet und eingeengt. Der Rückstand wird an Kieselgel (Laufmittel : Dichlormethan/Essigester 10:1) chromatografiert. Zur weiteren Reinigung wird aus Essigester umkristallisiert.

Fp. 140-145 °C ; H-NMR: 0,59 (2H, m, CH₂-bridge) 1,02 (3H, d, J= 8 Hz, H-7) 1,03 (3H, s, H-18) 1,12 (3H, s, H-19) 3,56 (1H, d J= 6 Hz, H-17) 5,68 (1H, m, H-4)

## Patentansprüche

1. Ungesättigte 14,15-Cyclopropano-Androstane
der allgemeinen Formel I 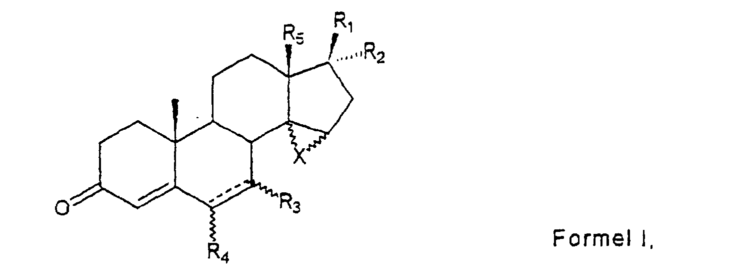 worin
R₁ ein Wasserstoffatom, eine Hydroxygruppe, eine Alkyloxy-, Acyloxy-, Aryloxy-, Aralkyloxy- oder eine Alkylaryloxygruppe , ein Rest -OCONHR₉ₐ oder -OCOOR₉ₐ
mit R₉ₐ für ein Wasserstoffatom , einen Alkyl-, Aryl- , Aralkyl- oder Alkylarylrest mit jeweils 1-10 Kohlenstoffatomen,
ist,
R₂ für ein Wasserstoffatom, eine Hydroxygruppe eine Alkyl-, Acyl-, Aryl-, Aralkyl-, Alkylarylgruppe mit jeweils 1-10 Kohlenstoffatomen,
für einen Rest -(CH₂)ₙCH₂Y
mit n = 0, 1 oder 2 und Y
für ein Fluor-,Chlor-, Brom-, oder Jodatom, für eine Cyano-, Azido- oder Rhodanogruppe,
für einen Rest -OR₁₀ oder -SR₁₀ mit R₁₀
für ein Wasserstoffatom, einen Alkyl-, Aryl-, Aralkyl- oder Alkylarylrest mit jeweils 1-10 Kohlenstoffatomen oder einen Acylrest COR_{9b}
mit R_{9b} für einen Alkyl-, Aryl-, Aralkyl- oder Alkylarylrest mit jeweils 1-10 Kohlenstoffatomen,
für einen Rest -OR₉ₐ
mit R₉ₐ für ein Wasserstoffatom, ein Alkyl-, Aryl-, Aralkyl- oder Alkylarylrest mit jeweils 1 - 10 Kohlenstoffatomen ,
für einen Rest - (CH₂)ₘ-CH=CH(CH₂)ₙ-R₈
mit m =0, 1, 2 oder 3 und n = 0, 1 oder 2 und R₈ für ein Wasserstoffatom, einen Alkyl-, Aryl-, Aralkyl- oder Alkylarylrest mit jeweils 1-10 Kohlenstoffatomen oder eine Hydroxylgruppe, eine Alkoxygruppe, einer Acyloxygruppe mit jeweils 1-10 Kohlenstoffatomen,
für einen Rest - (CH₂)ₒC≡CR₁₁
mit o = 0,1 oder 2 und R₁₁ für ein Wasserstoffatom, ein Fluor-, Chlor-, Brom- oder lodatom, einen Alkyl-, Aryl-, Aralkyl-Alkylaryl-, oder Acylrest mit jeweils 1-10 Kohlenstoffatomen,
R₁ und R₂ für eine Keto-, Methylen-, Difluormethylengruppe stehen,
sich zwischen C- 6 und C-7 eine Doppelbindung befinden kann,
sich zwischen C-14 und C-15 eine α- oder β-Cyclopropangruppe X befindet mit X
für eine CZ₂- Gruppe mit Z für ein Wasserstoff-, Fluor-, Chlor-, Brom- oder lodatom,
R₃ und R₄ unabhängig voneinander für ein Wasserstoffatom, eine α- oder β- standige Alkylgruppe mit 1-10 Kohlensstoffatomen stehen
R₅ für eine Alkylgruppe mit 1-3 Kohlenstoffatomen steht.

2. Verbindungen nach Anspruch 1, nämlich
17β-Hydroxy-14α,15α-methylen-androst-4-en-3-on (J 1193),
17α-Hydroxy-14α,15α-methylen-androst-4-en-3-on,
17β-Hydroxy-14β,15β-methylen-androst-4-en-3-on,
17α-Hydroxy-14β,15β-methylen-androst-4-en-3-on,
17α-Methyl,17β-Hydroxy-14α,15α-methylen-androst-4-en-3-on,
17β-Methyl,17α-Hydroxy-14α,15α-methylen-androst-4-en-3-on,
17α-Methyl,17β-Hydroxy-14β,15β-methylen-androst-4-en-3-on,
17β-Methyl,17α-Hydroxy-14β,15β-methylen-androst-4-en-3-on,
17β-Hydroxy-6α-Methyl-14α,15α-methylen-androst-4-en-3-on,
17α-Hydroxy-6α-Methyl-14α,15α-methylen-androst-4-en-3-on,
17β-Hydroxy-6α-Methyl-14β,15β-methylen-androst-4-en-3-on,
17α-Hydroxy-6α-Methyl-14β,15β-methylen-androst-4-en-3-on,
17β-Hydroxy-7α-Methyl-14α,15α-methylen-androst-4-en-3-on,
17α-Hydroxy-7α-Methyl-14α,15α-methylen-androst-4-en-3-on,
17β-Hydroxy-7α-Methyl-14β,15β-methylen-androst-4-en-3-on,
17α-Hydroxy-7α-Methyl-14β,15β-methylen-androst-4-en-3-on,
17β-Hydroxy-14α,15α-methylen-androsta-4,6-dien-3-on,
17α-Hydroxy-14α,15α-methylen-androsta-4,6-dien-3-on,
17β-Hydroxy-14β,15β-methylen-androsta-4,6-dien-3-on,
17α-Hydroxy-14β,15β-methylen-androsta-4,6-dien-3-on,
17α-Methyl,17β-Hydroxy-14α,15α-methylen-androsta-4,6-dien-3-on,
17β-Methyl,17α-Hydroxy-14α,15α-methylen-androsta-4,6-dien-3-on,
17α-Methyl;17β-Hydroxy-14β,15β-methylen-androsta-4,6-dien-3-on,
17β-Methyl,17α-Hydroxy-14β,15β-methylen-androsta-4,6-dien-3-on,
17β-Hydroxy-7α,17α-Dimethyl-14α,15α-methylen-androst-4-en-3-on,
17α-Hydroxy-7α,17α-Dimethyl-14α,15α-methylen-androst-4-en-3-on,
17β-Hydroxy-7α,17α-Dimethyl-14β,15β-methylen-androst-4-en-3-on,
17α-Hydroxy-7α,17β-Dimethyl-14β,15β-methylen-androst-4-en-3-on,

3. Verfahren zur Herstellung der Verbindungen nach Anspruch 1 und deren pharmazeutisch annehmbaren Salzen,
**dadurch gekennzeichnet,**
**daß** man in Verbindungen der allgemeinen Formel II, 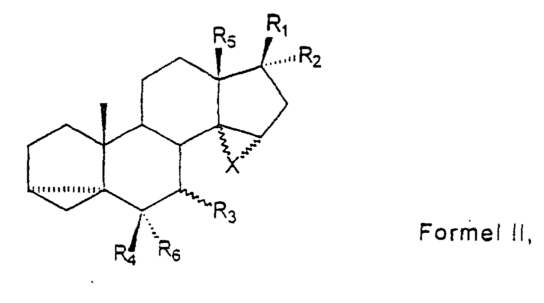
worin R₁, R₂ , R₃ und R₅ die in Anspruch 1 angegebene Bedeutung besitzen,
sich zwischen C-14 und C-15 eine α- oder β-Cyclopropangruppe X befindet mit X
für eine CZ2- Gruppe mit Z für ein Wasserstoff-, Fluor-, Chlor-, Brom- oder lodatom,
R₄ für ein Wasserstoffatom, eine Hydroxygruppe , eine Alkoxylgruppe , eine Acyloxygruppe, eine Alkylgruppe mit jeweils 1-10 Kohlenstoffatomen steht,
R₆ für ein Wasserstoffatom, eine Hydroxygruppe , eine Alkoxylgruppe , eine Acyloxygruppe , eine Alkylgruppe mit jeweils 1-10 Kohlenstoffatomen steht,
die 3,5-Cyclopropangruppierung mittels organischer Säuren und Lewissäuren spaltet
und in an sich bekannter Weise in die Verbindungen der allgemeinen Formel I überführt.

4. Pharmazeutische Zusammensetzung, die mindestens eine Verbindung der allgemeinen Formel (I) nach einem der Ansprüche 1 und 2 oder deren Säureadditionssalz, zusammen mit Verdünnungsmitteln und/oder Trägerstoffen enthält.

5. Verwendung der Verbindungen der allgemeinen Formel (I) nach einem der Ansprüche 1 und 2 zur Herstellung eines Arzneimittels zur Fertilitätskontrolle, der Hormon-Replacement-Therapie (HRT) oder der Behandlung hormonell bedingter Erkrankungen, wie beispielsweise Endometriose, Mammacarcinom oder Hypogonadismus.

6. Verbindungen der allgemeinen Formel (I) nach einem der Ansprüche 1 und 2 zur Anwendung als therapeutische Wirkstoffe.

## Claims

1. Unsaturated l4,15-cyclopropanoandrostanes of the general formula I 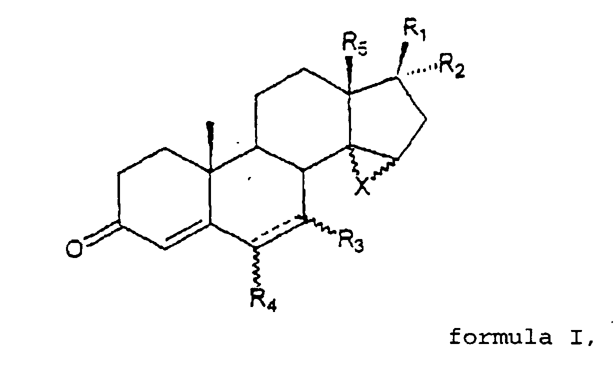 in which
R₁ is a hydrogen atom, a hydroxyl group, an alkyloxy, acyloxy, aryloxy, aralkyloxy or an alkylaryloxy group, a radical -OCONHR₉ₐ or - OCOOR₉ₐ
where R₉ₐ is a hydrogen atom, an alkyl, aryl, aralkyl or alkylaryl radical in each case having 1-10 carbon atoms,
R₂ is a hydrogen atom, a hydroxyl group, an alkyl, acyl, aryl, aralkyl or alkylaryl group in each case having 1-10 carbon atoms,
a radical -(CH₂)ₙCH₂Y
where n = 0, 1 or 2 and Y
is a fluorine, chlorine, bromine or iodine atom, a cyano, azido or thiocyanato group,
a radical -OR₁₀ or -SR₁₀ where R₁₀ is a hydrogen atom,
an alkyl, aryl, aralkyl or alkylaryl radical in each case having 1-10 carbon atoms or an acyl radical COR_{9b}
where R_{9b} is an alkyl, aryl, aralkyl or alkylaryl radical in each case having 1-10 carbon atoms,
a radical -OR₉ₐ
where R₉ₐ is a hydrogen atom, an alkyl, aryl, aralkyl or alkylaryl radical in each case having 1-10 carbon atoms,
a radical -(CH₂)ₘ-CH=CH(CH₂)ₙ-R₈
where m = 0, 1, 2 or 3 and n = 0, 1 or 2 and R₈ is a hydrogen atom, an alkyl, aryl, aralkyl or alkylaryl radical in each case having 1-10 carbon atoms or a hydroxyl group, an alkoxy group, an acyloxy group in each case having 1-10 carbon atoms,
a radical (CH₂)ₒC≡CR₁₁
where o = 0, 1 or 2 and R₁₁ is a hydrogen atom, a fluorine, chlorine, bromine or iodine atom, an alkyl, aryl, aralkyl, alkylaryl or acyl radical in each case having 1-10 carbon atoms,
R₁ and R₂ are a keto, methylene or difluoromethylene group,
a double bond can be located between C-6 and C-7,
an α- or β-cyclopropane group X can be located between C-14 and C-15 where X
is a CZ₂ group where Z is a hydrogen, fluorine, chlorine, bromine or iodine atom,
R₃ and R₄ independently of one another are a hydrogen atom, an α- or β-alkyl group having 1-10 carbon atoms,
R₅ is an alkyl group having 1-3 carbon atoms.

2. Compounds according to Claim 1,
namely
17β-hydroxy-14α,15α-methyleneandrost-4-en-3-one (J 1193),
17α-hydroxy-14α,15α-methyleneandrost-4-en-3-one,
17β-hydroxy-14β,15β-methyleneandrost-4-en-3-one,
17α-hydroxy-14β,15β-methyleneandrost-4-en-3-one,
17α-methyl,17β-hydroxy-14α,15α-methyleneandrost,-4-en-3-one,
17β-methyl,17α-hydroxy-14α,15α-methyleneandrost-4-en-3-one,
17α-methyl,17β-hydroxy-14β,15β-methyleneandrost-4-en-3-one,
17β-methyl,17α-hydroxy-14β,15β-methyleneandrost-4-en-3-one,
17β-hydroxy-6α-methyl-14α,15α-methyleneandrost,4-en-3-one,
17α-hydroxy-6α-methyl-14α,15α-methyleneandrost-4-en-3-one,
17β-hydroxy-6α-methyl-14β,15β-methyleneandrost-4-en-3-one,
17α-hydroxy-6α-methyl-14β,15β-methyleneandrost-4-en-3-one,
17β-hydroxy-7α-methyl-14α,15α-methyleneandrost-4-en-3-one,
17α-hydroxy-7α-methyl-14α,15α-methyleneandrost-4-en-3-one,
17β-hydroxy-7α-methyl-14β,15β-methyleneandrost-4-en-3-one,
17α-hydroxy-7α-methyl-14β,15β-methyleneandrost-4-en-3-one,
17β-hydroxy-14α,15α-methyleneandrosta-4,6-dien-3-one,
17α-hydroxy-14α,15α-methyleneandrosta-4,6-dien-3-one,
17β-hydroxy-14β,15β-methyleneandrosta-4,6-dien-3-one,
17α-hydroxy-14β,15β-methyleneandrosta-4,6-dien-3-one,
17α-methyl,17β-hydroxy-14α,15α-methyleneandrosta-4,6-dien-3-one,
17β-methyl,17α-hydroxy-14α,15α-methyleneandrosta-4,6-dien-3-one,
17α-methyl,17β-hydroxy-14β,15β-methyleneandrosta-4,6-dien-3-one,
17β-methyl,17α-hydroxy-14β,15β-methyleneandrosta-4,6-dien-3-one,
17β-hydroxy-7α,17α-dimethyl-14α,15α-methylene-androst-4-en-3-one,
17α-hydroxy-7α,17β-dimethyl-14α,15α-methylene-androst-4-en-3-one,
17β-hydroxy-7α,17α-dimethyl-14β,15β-methylene-androst-4-en-3-one,
17α-hydroxy-7α,17β-dimethyl-14β,15β-methylene-androst-4-en-3-one.

3. Process for the preparation of the compounds according to Claim 1 and their pharmaceutically acceptable sals,
**characterized in that**,
in compounds of the general formula II, 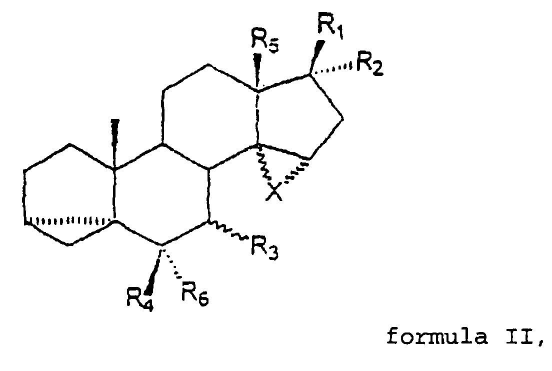
in which R₁, R₂, R₃ and R₅ have the meaning indicated in Claim 1,
an α- or β-cyclopropane group X is located between C-14 and C-15 where X
is a CZ₂ group where Z is a hydrogen, fluorine, chlorine, bromine or iodine atom,
R₄ is a hydrogen atom, a hydroxyl group, an alkoxy group, an acyloxy group, an alkyl group in each case having 1-10 carbon atoms,
R₆ is a hydrogen atom, a hydroxyl group, an alkoxy group, an acyloxy group, an alkyl group in each case having 1-10 carbon atoms,
the 3,5-cyclopropane group is cleaved by means of organic acids and Lewis acids
and they are converted into the compounds of the general formula I in a manner known per se.

4. Pharmaceutical composition which contains at least one compound of the general formula (I) according to one of Claims 1 and 2 or its acid addition salt, together with diluents and/or vehicles.

5. Use of the compounds of the general formula (I) according to one of Claims 1 and 2 for the production of a medicament for fertility control, hormone replacement therapy (HRT) or the treatment of hormonally-related disorders, such as, for example, endometriosis, breast carcinoma or hypogonadism.

6. Compounds of the general formula (I) according to one of Claims 1 and 2 for use as therapeutic active compounds.

## Revendications

1. 14,15-cyclopropano-androstanes insaturés de formule générale I 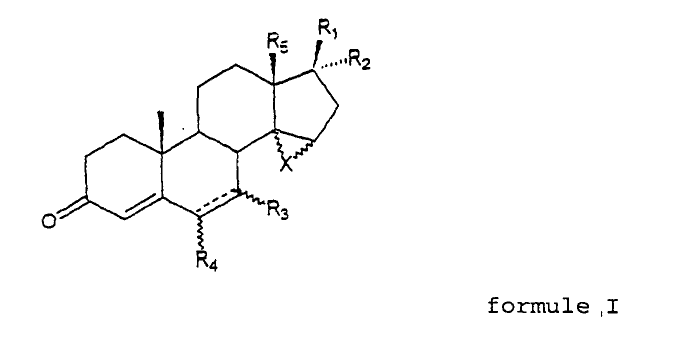 dans laquelle
R₁ est un atome d'hydrogène, un groupe hydroxy, un groupe alkyloxy, acyloxy, aryloxy, aralkyloxy ou alkylaryloxy, un radical -OCONHR₉ₐ ou -OCOOR₉ₐ
R₉ₐ représentant un atome d'hydrogène, un radical alkyle, aryle, aralkyle ou alkylaryle ayant chacun de 1 à 10 atomes de carbone,
R₂ représente un atome d'hydrogène, un groupe hydroxy, un groupe alkyle, acyle, aryle, aralkyle, alkylaryle ayant chacun de 1 à 10 atomes de carbone,
un radical -(CH₂)ₙCH₂Y,
n étant égal à 0, 1 ou 2 et
Y représentant un atome de fluor, chlore, brome ou iode, un groupe cyano, azido ou sulfocyano,
un radical -OR₁₀ ou -SR₁₀,
R₁₀ représentant un atome d'hydrogène,
un radical alkyle, aryle, aralkyle ou alkylaryle ayant chacun de 1 à 10 atomes de carbone,
ou un radical acyle COR_{9b,}
R_{9b} représentant un radical alkyle, aryle, aralkyle ou alkylaryle ayant chacun de 1 à 10 atomes de carbone,
un radical -OR₉ₐ
R₉ₐ représentant un atome d'hydrogène, un radical alkyle, aryle, aralkyle ou alkylaryle ayant chacun de 1 à 10 atomes de carbone,
un radical -(CH₂)ₘ-CH=CH(CH₂)ₙ-R₈,
où m = 0, 1, 2 ou 3 et n = 0, 1 ou 2
et R₈ représente un atome d'hydrogène, un radical alkyle, aryle, aralkyle ou alkylaryle ayant chacun de 1 à 10 atomes de carbone, ou
un groupe hydroxy, alcoxy, acyloxy ayant chacun de 1 à 10 atomes de carbone,
un radical -(CH₂)ₒC≡CR₁₁,
où O = 0, 1 ou 2 et
R₁₁ représente un atome d'hydrogène, un atome de fluor, chlore, brome ou iode, un radical alkyle, aryle, aralkyle, alkylaryle ou acyle ayant chacun de 1 à 10 atomes de carbone, où
R₁ et R₂ représentent un groupe céto, méthylène, difluorométhylène,
une double liaison peut être présente entre C-6 et C-7,
entre C-14 et C-15 est présent un groupe α- ou β-cyclopropane X, où X
représente un groupe CZ₂, Z représentant un atome d'hydrogène, de fluor, chlore, brome ou iode,
R₃ et R₄ représentent, indépendamment l'un de l'autre, un atome d'hydrogène, un groupe alkyle en position α ou β, ayant de 1 à 10 atomes de carbone,
R₅ représente un groupe alkyle ayant de 1 à 3 atomes de carbone.

2. Composés selon la revendication 1,
à savoir
17β-hydroxy-14α,15α-méthylène-androst-4-én-3-one (J 1193),
17α-hydroxy-14α,15α-méthylène-androst-4-én-3-one,
17β-hydroxy-14β,15β-méthylène-androst-4-én-3-one,
17α-hydroxy-14β,15β-méthylène-androst-4-én-3-one,
17α-méthyl,17β-hydroxy-14α,15α-méthylène-androst-4-én-3-one,
17β-méthyl,17α-hydroxy-14α,15α-méthylène-androst-4-én-3-one,
17α-méthyl,17β-hydroxy-14β,15β-méthylène-androst-4-én-3-one,
17β-méthyl,17α-hydroxy-14β,15β-méthylène-androst-4-én-3-one,
17β-hydroxy-6α-méthyl-14α,15α-méthylène-androst-4-én-3-one,
17α-hydroxy-6α-méthyl-14α,15α-méthylène-androst-4-én-3-one,
17β-hydroxy-6α-méthyl-14β,15β-méthylène-androst-4-én-3-one,
17α-hydroxy-6α-méthyl-14β,15β-méthylène-androst-4-én-3-one,
17β-hydroxy-7α-méthyl-14α,15α-méthylène-androst-4-én-3-one,
17α-hydroxy-7α-méthyl-14α,15α-méthylène-androst-4-én-3-one,
17β-hydroxy-7α-méthyl-14β,15β-méthylène-androst-4-én-3-one,
17α-hydroxy-7α-méthyl-14β,15β-méthylène-androst-4-én-3-one,
17β-hydroxy-14α,15α-méthylène-androsta-4,6-dién-3-one,
17α-hydroxy-14α,15α-méthylène-androsta-4,6-dién-3-one,
17β-hydroxy-14β,15β-méthylène-androsta-4,6-dién-3-one,
17α-hydroxy-14β,15β-méthylène-androsta-4,6-dién-3-one,
17α-méthyl,17β-hydroxy-14α,15α-méthylène-androsta-4,6-dién-3-one,
17β-méthyl,17α-hydroxy-14α,15α-méthylène-androsta-4,6-dién-3-one,
17α-méthyl,17β-hydroxy-14β,15β-méthylène-androsta-4,6-dién-3-one,
17β-méthyl,17α-hydroxy-14β,15β-méthylène-androsta-4,6-dién-3-one,
17β-hydroxy-7α,17α-diméthyl-14α,15α-méthylène-androst-4-én-3-one,
17α-hydroxy-7α,17β-diméthyl-14α,15α-méthylène-androst-4-én-3-one,
17β-hydroxy-7α,17α-diméthyl-14β,15β-méthylène-androst-4-én-3-one,
17α-hydroxy-7α,17β-diméthyl-14β,15β-méthylène-androst-4-én-3-one.

3. Procédé pour la préparation des composés selon la revendication 1 et de leurs sels pharmaceutiquement acceptables, **caractérisé en ce que**, dans des composés de formule générale II, 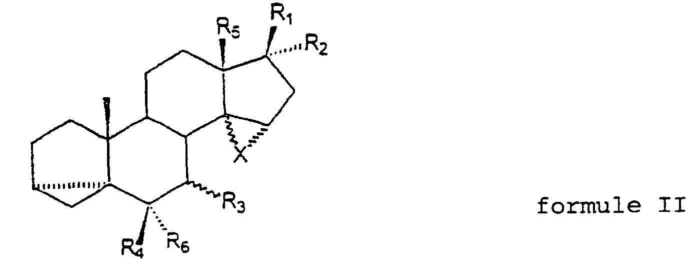
dans laquelle R₁, R₂, R₃ et R₅ ont les significations données dans la revendication 1,
entre C-14 et C-15 est présent un groupe α- ou β-cyclopropane X, où X
représente un groupe CZ₂, Z représentant un atome d'hydrogène, de fluor, chlore, brome ou iode,
R₄ représente un atome d'hydrogène, un groupe hydroxy, un groupe alcoxy, un groupe acyloxy, un groupe alkyle ayant chacun de 1 à 10 atomes de carbone,
R₆ représente un atome d'hydrogène, un groupe hydroxy, un groupe alcoxy, un groupe acyloxy, un groupe alkyle ayant chacun de 1 à 10 atomes de carbone,
on ouvre le groupement 3,5-cyclopropane au moyen d'acides organiques et d'acides de Lewis
et on les convertit d'une façon connue en soi en les composés de formule générale I.

4. Composition pharmaceutique qui contient au moins un composé de formule générale (I) selon l'une quelconque des revendications 1 et 2 ou un sel d'addition avec un acide de celui-ci, conjointement avec des diluants et/ou véhicules.

5. Utilisation des composés de formule générale (I) selon l'une quelconque des revendications 1 et 2, pour la fabrication d'un médicament destiné à la régulation de la fertilité, à l'hormonothérapie substitutive (HRT) ou au traitement de maladies d'origine hormonale, comme par exemple l'endométriose, le cancer du sein ou l'hypogonadisme.

6. Composés de formule générale (I) selon l'une quelconque des revendications 1 et 2, pour utilisation en tant que substances actives thérapeutiques.
